# EUROPEAN PATENT SPECIFICATION

(11) **EP 4 252 693 B1**
(45) Date of publication and mention of the grant of the patent: **29.04.2026**
(21) Application number: 20965042.3
(22) Date of filing: 08.12.2020
(51) Int. Cl.: A61B 34/00, A61B 17/30, A61B 17/28, A61B 17/29, A61B 34/30, A61B 17/00

(54) **FORCEPS DEVICE AND BASE COMPONENT**
ZANGENVORRICHTUNG UND BASISKOMPONENTE
DISPOSITIF DE PINCE ET COMPOSANT DE BASE

(43) Date of publication of application: 04.10.2023
(73) Proprietor: RIVERFIELD Inc., 1070052 Tokyo (JP)
(72) Inventor: TAKIKAWA, Kyohei, Tokyo 160-0017 (JP)
(74) Representative: Adares PartGmbB
(86) International application number: PCT/JP2020/045719
(87) International publication number: WO 2022/123658

(56) References cited:
- EP-A1- 3 845 155
- WO-A1-2020/044994
- CN-A- 108 430 374
- JP-A- 2019 501 699
- JP-A- H10 179 601
- US-A- 5 810 876
- US-A- 5 908 437
- US-A1- 2011 125 166
- US-A1- 2015 127 045
- US-A1- 2019 099 231
- US-A1- 2020 000 533
- US-A1- 2020 138 507

## Description

### Technical Field

The present invention relates to a forceps device used for a manipulator of a surgical robot.

### Background Art

Medical treatments using robots (manipulators) have recently been proposed in order to reduce the burden on operators and save manpower in medical facilities. In the field of surgery, proposals have been made for surgical manipulator systems for operators to treat patients by operating remotely-controllable surgical manipulators.

Various surgical tools are attached to leading ends of surgical manipulators. Known surgical tools include forceps for grasping human tissue during surgery. For example, Patent Literature 1 teaches forceps including two jaw parts serving as grasping portions, two disks having different outer diameters from each other provided on the respective jaw parts, and wires running over the disks. The wires are pulled to open or close the jaw parts.

Patent Literature 2 describes a forceps device having an end effector which is coupled to a shaft via hinge and including joints for rotating the end effector.

Patent Literature 3 describes a surgical tool having a drive housing and an elongate shaft extending from the drive housing. A wrist couples an end effector to the elongate shaft. A plurality of drive cables extends between the drive housing and the end effector, and movement of the drive cables causes the end effector to articulate about an pivot axis.

Patent Literature 4 describes a surgical tool comprising cutting jaws which are closed and opened by a link mechanism having levers having holes for passing wires and which is manipulated by a manipulator having a wire.

Patent Literature 5 describes a surgical tool comprising a wrist having segments or disks and a plurality of cables or wires used to bend the wrist in pitch and yaw.

Patent Literature 6 describes a surgical instrument comprising elongate elements, an end effector which can be grasping forceps, a first support body, a second support body, a silicone seal, a pulley group, and a shaft, wherein the pulley group includes a first pulley group, a second pulley group, and a third pulley group.

Patent Literature 7 describes a forceps device having an elastomeric diaphragm having two holes whose diameters are slightly smaller than the diameters of control wires and acting as a sealing means.

### Related Art List

### Patent Literature

Patent Literature 1: US 2015/0127045 A1
Patent Literature 2: CN 108 430 374 A
Patent Literature 3: US 2019/099231 A1
Patent Literature 4: US 5 908 437 A
Patent Literature 5: US 2011/125166 A1
Patent Literature 6: WO 2020/044994 A1
Patent Literature 7: US 5 810 876 A

### Summary of Invention

### Technical Problem

When an operation is performed with the aforementioned forceps inserted into a patient's abdomen, the air pressure inside the abdominal cavity may be increased to be higher than the atmospheric pressure in order to secure the surgical field. If, however, gas inside the abdominal cavity leaks out of the body through a leading end of the forceps and a shaft, the air pressure inside the abdominal cavity cannot be increased to be higher than the atmospheric pressure. In particular, in a case where the jaw parts at the leading end of the forceps are opened and closed by forward and backward movements of wires running through the shaft, sealing based on consideration of the wire movements is necessary.

The present invention has been made in view of the aforementioned circumstances, and an exemplary object thereof is to provide a novel technology for improving the airtightness of the grasping part side of a forceps device.

### Solution to problem

To solve the aforementioned problems, a forceps device according to an aspect of the present invention includes: a grasping part; a support that holds the grasping part; a rotating shaft that turnably supports the support; a base member that holds the rotating shaft; a plurality of grasping-portion wires for transmitting driving forces to move the grasping part; a guide pulley for guiding one or some of the plurality of grasping-portion wires; and a support shaft for rotatably supporting the guide pulley. The base member includes: a partition part having a plurality of grasping-portion-wire holes through which the respective grasping-portion wires pass; and a pair of arms extending from the partition part toward the grasping part, respective ends of the support shaft being fixed to the respective arms. The plurality of grasping-portion wires includes a first grasping-portion wire and a second grasping-portion wire. The plurality of grasping-portion-wire holes includes a first grasping-portion-wire hole through which the first grasping-portion wire passes, and a second grasping-portion-wire hole which is closest to the first grasping-portion-wire hole and through which the second grasping-portion wire passes. The guide pulley guides the one or some of the plurality of grasping-portion wires so that the first grasping-portion wire is separated from the second grasping-portion wire.

According to this aspect, the first grasping-portion-wire hole can be separated from the second grasping-portion-wire hole. This allows a sealing member having holes to be molded with high accuracy on one side of the partition part having the holes and increases the airtightness on the grasping part side, for example.

When the partition part is viewed in a vertical direction, an angle at a center of the partition part between the first grasping-portion-wire hole and the second grasping-portion-wire hole may be equal to or larger than 30°. As a result, the distance between the first grasping-portion-wire hole and the second grasping-portion-wire hole becomes large, which reduces such defects as integration of holes when the sealing member with a plurality of holes is molded, for example.

The base member may include a sealing member on one side with respect to the partition part, and the sealing member may have a plurality of sealing holes at positions corresponding to the plurality of grasping-portion-wire holes. This enables sealing with the sealing member with the grasping-portion wires passing through the base member.

The sealing holes each have a diameter smaller than a diameter of a corresponding one of the grasping-portion-wire holes and smaller than a diameter of a corresponding one of the grasping-portion wires. As a result, even when a gap is present between a grasping-portion-wire hole and a grasping-portion wire, a sealing hole and the grasping-portion wire are in close contact with each other, which reduces leakage of gas from the grasping part side to the outside of the forceps device via the base member.

The sealing member may be a silicone resin film.

A pair of support wires for transmitting driving forces to turn the support may further be included. The partition part may have a pair of support-wire holes through which the pair of support wires, respectively, pass, and when the partition part is viewed in a vertical direction, an angle at a center of the partition part between each of the support-wire holes and the grasping-portion-wire hole that is closest to the support-wire hole may be equal to or larger than 30°. As a result, the support-wire holes and the grasping-portion-wire holes can be separated from each other. Thus, the sealing member having holes is molded with high accuracy on one side with respect to the partition part having holes, and the airtightness on the grasping part side is increased, for example.

A non-inventive aspect is a base member. The base member is arranged between a grasping part and a shaft of a forceps device. The base member includes a partition part having a plurality of grasping-portion-wire holes through which a plurality of grasping-portion wires, respectively, pass, the grasping-portion wires transmitting driving forces to move the grasping part. The plurality of grasping-portion-wire holes include a first grasping-portion-wire hole and a second grasping-portion-wire hole which is closest to the first grasping-portion-wire hole. When the partition part is viewed in a vertical direction, an angle at a center of the partition part between the first grasping-portion-wire hole and the second grasping-portion-wire hole is equal to or larger than 30°.

According to this aspect, the first grasping-portion-wire hole and the second grasping-portion-wire hole can be widely separated from each other. This allows a sealing member having holes to be molded with high accuracy on one side of the partition part having the holes and increases the airtightness on the grasping part side, for example. In addition, such defects as integration of holes when the sealing member with a plurality of holes is molded, for example, can be reduced.

The partition part may have a pair of support-wire holes through which a pair of support wires, respectively, pass, the support wires transmitting driving forces for turning a support that holds the grasping part, and when the partition part is viewed in the vertical direction, an angle at the center of the partition part between each of the support-wire holes and the grasping-portion-wire hole that is closest to the support-wire hole may be equal to or larger than 30°. As a result, the support-wire holes and the grasping-portion-wire holes can be separated from each other. Thus, the sealing member having holes is molded with high accuracy on one side with respect to the partition part having holes, and the airtightness on the grasping part side is increased, for example.

A pair of arms extending from the partition part toward the grasping part may further be included. The arms may each have a fitting hole, respective ends of a support shaft that rotatably supports a pair of guide pulleys being fitted into the fitting holes. As a result, the pair of arms are fixed via the support shaft, which increases the stiffness of the base member.

### Advantageous Effects of Invention

According to the present invention, a forceps device with high airtightness on the grasping part side can be achieved.

### Brief Description of Drawings

FIG. 1 is a perspective view of a forceps device according to an embodiment.
FIG. 2 is a front view of the forceps device of FIG. 1 as viewed in a direction A.
FIG. 3 is a side view of the forceps device of FIG. 1 as viewed in a direction B.
FIG. 4 is a side view of the forceps device of FIG. 1 as viewed in a direction C.
FIG. 5 is a cross-sectional view of the forceps device illustrated in FIG. 3 along D-D.
FIG. 6 is an enlarged view of a region R in FIG. 5.
FIG. 7 is a front view of a guide pulley according to the embodiment.
FIG. 8 is a cross-sectional view of the guide pulley illustrated in FIG. 7 along E-E.
FIG. 9 is a perspective view of a support according to the embodiment.
FIG. 10 is a flowchart explaining a method for manufacturing the forceps device according to the embodiment.
FIG. 11 is a perspective view illustrating a state in which a pair of jaw parts facing each other are to be fitted according to the embodiment.
FIG. 12 is a perspective view of the forceps device illustrated in FIG. 1 as viewed in a direction H1.
FIG. 13 is a perspective view of the forceps device illustrated in FIG. 1 as viewed in a direction H2.
FIG. 14 is a perspective view of the forceps device illustrated in FIG. 1 as viewed in a direction H3.
FIG. 15 is a perspective view of the forceps device illustrated in FIG. 1 as viewed in a direction opposite the direction H2.
FIG. 16 is a view of the forceps device illustrated in FIG. 3 without illustration of a base member.
FIG. 17 is a view of the forceps device illustrated in FIG. 4 without illustration of the base member.
FIG. 18 is a view of the forceps device illustrated in FIG. 1 without illustration of wires.
FIG. 19 is a perspective view of the base member according to the embodiment.
FIG. 20 is a top view of the base member illustrated in FIG. 19 as viewed in a direction J1.
FIG. 21 is a bottom view of the base member illustrated in FIG. 19 as viewed in a direction J2.
FIG. 22 is a perspective view of a jig to be used for producing a sealing member.
FIG. 23 is a perspective view illustrating a state in which the jig illustrated in FIG. 22 is attached to the base member.
FIG. 24 is a schematic diagram illustrating injection of sealing resin in a state in which the jig is attached to the base member.

### Description of Embodiments

The present invention will now be described on the basis of an embodiment with reference to the drawings. Components, members, and processes that are the same as or equivalent to each other illustrated in the drawings are represented by the same reference numerals, and redundant explanation will not be repeated where appropriate. The embodiment is not to limit the invention, but is an example, and any feature or any combination of features described in the embodiment is not necessarily essential to the invention.

The invention is defined by the appended claims.

### 5 [Forceps device]

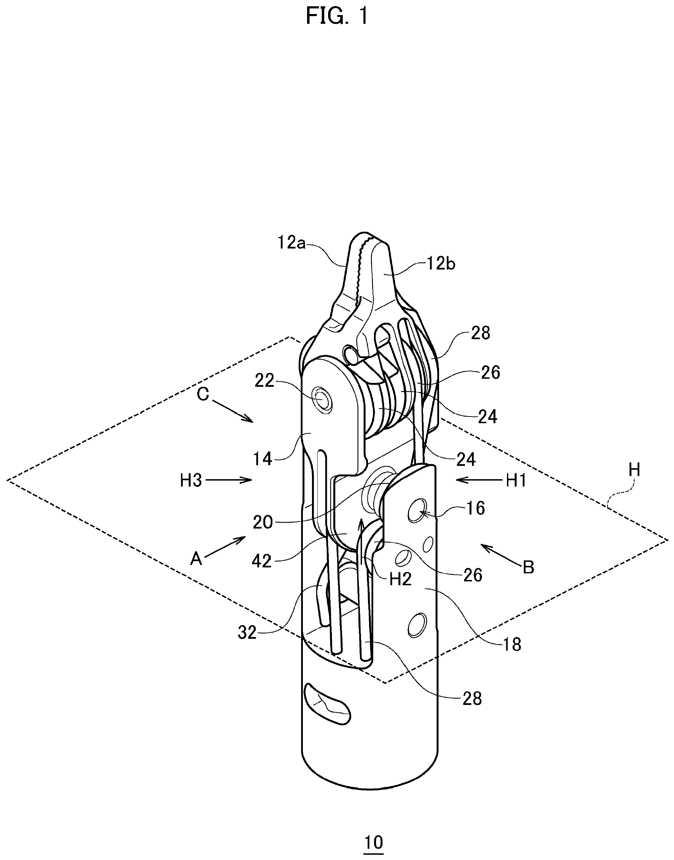

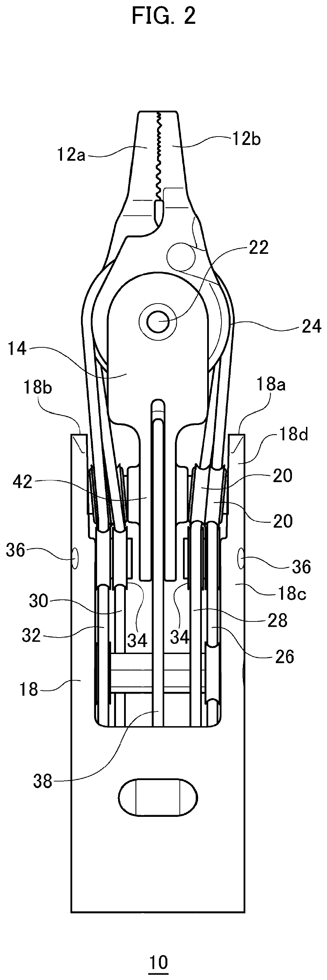

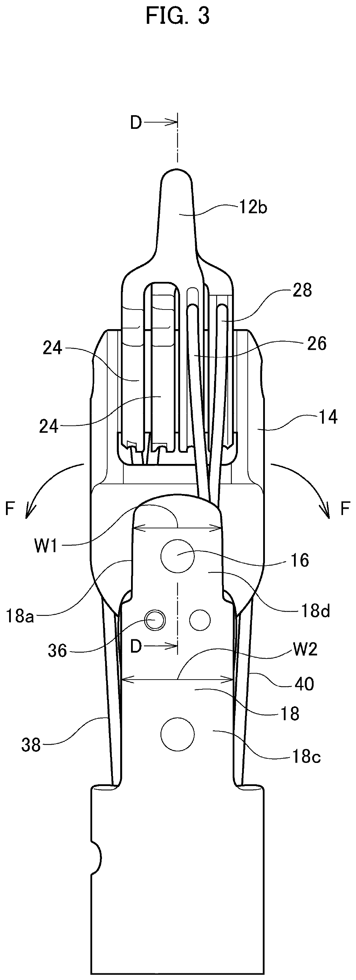

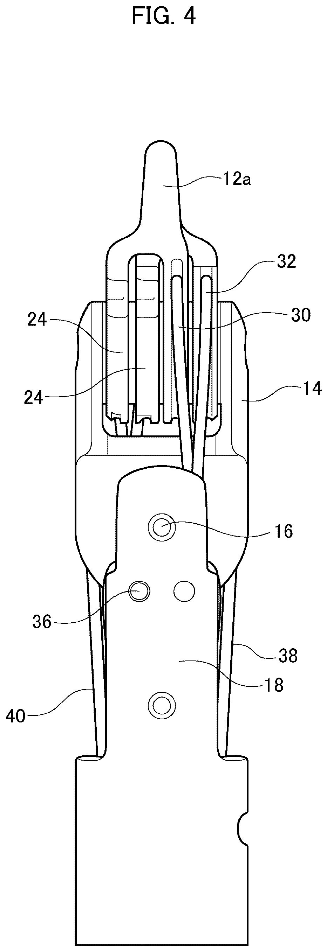

FIG. 1 is a perspective view of a forceps device according to an embodiment. FIG. 2 is a front view of the forceps device of FIG. 1 as viewed in a direction A. FIG. 3 is a side view of the forceps device of FIG. 1 as viewed in a direction B. FIG. 4 is a side view of the forceps device of FIG. 1 as viewed in a direction C.

The forceps device 10 illustrated in the drawings includes a pair of grasping portions 12a and 12b, a support 14 that holds the pair of grasping portions 12a and 12b, a first rotating shaft 16 that turnably supports the support 14, a base member 18 that holds the first rotating shaft 16, four guide pulleys 20 arranged coaxially with the first rotating shaft 16, a second rotating shaft 22 that turnably supports the pair of grasping portions 12a and 12b and is held by the support 14, four jaw pulleys 24 supported coaxially with the second rotating shaft 22, four wires 26, 28, 30 and 32 running over the four guide pulleys 20 and the four jaw pulleys 24, and wires 38 and 40 for rotating the support 14 about the first rotating shaft 16.

### [Guide pulleys 20]

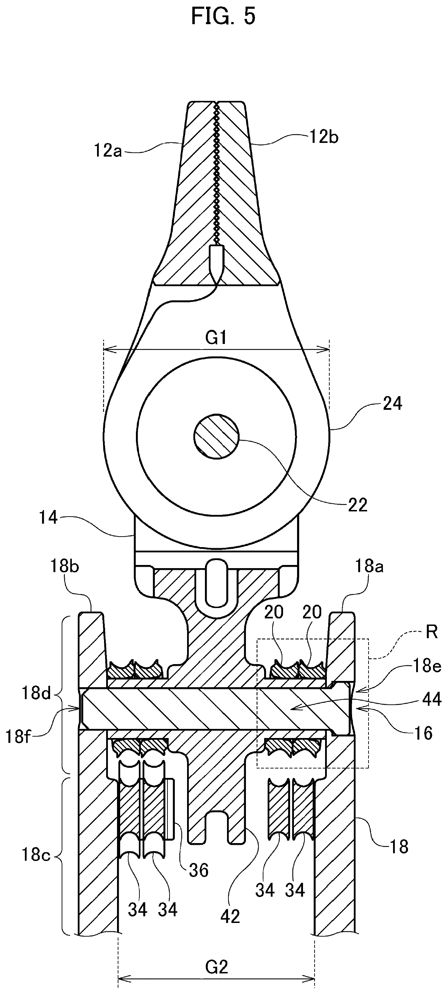

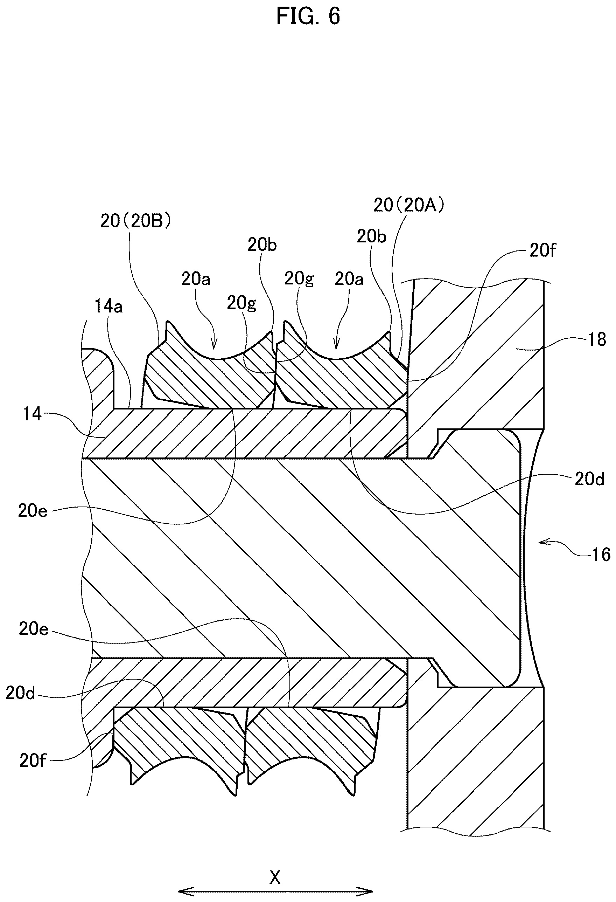

FIG. 5 is a cross-sectional view of the forceps device 10 illustrated in FIG. 3 along D-D. FIG. 6 is an enlarged view of a region R in FIG. 5. As illustrated in FIGS. 5 and 6, the guide pulleys 20 are rotatably supported by the first rotating shaft 16 with an annular part 14a having a cylindrical shape, which is part of the support 14, therebetween and at an angle with respect to the first rotating shaft 16. Thus, the respective wires 26, 28, 30 and 32 have small substantial fleet angles with respect to the guide pulleys 20, and therefore become less likely to interfere with edges 20b of guide grooves 20a of the guide pulleys 20. Alternatively, the guide pulleys 20 may be directly supported by the first rotating shaft 16 without the support 14 therebetween.

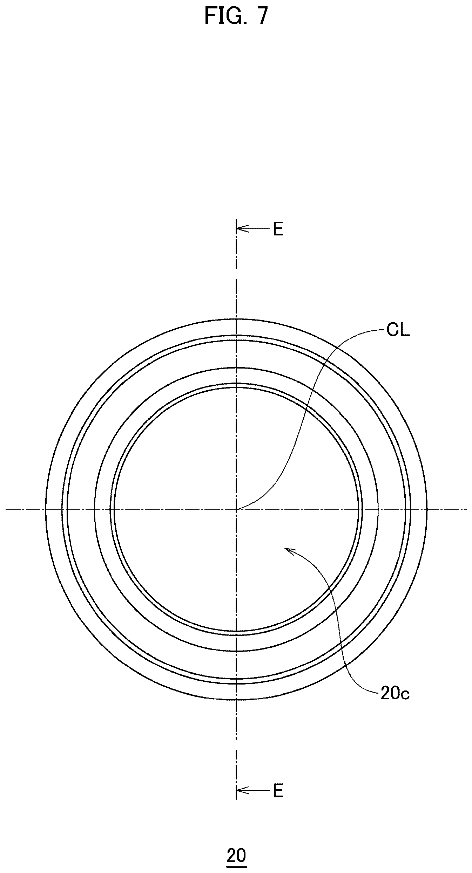

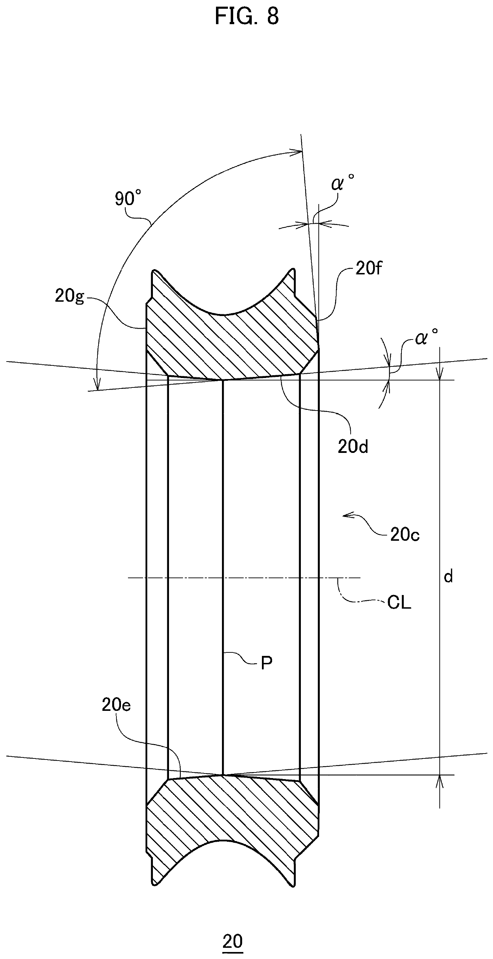

FIG. 7 is a front view of a guide pulley 20 according to the embodiment. FIG. 8 is a cross-sectional view of the guide pulley 20 illustrated in FIG. 7 along E-E. Each guide pulley 20 according to the embodiment has an inner circumferential face 20d tilted with respect to a center line CL of a circular opening 20c thereof through which the first rotating shaft 16 extends. The inner circumferential face 20d slides relative to the annular part 14a of the support 14 into which the first rotating shaft 16 is inserted. Thus, the guide pulleys 20 are autonomously tilted due to the forces received from wires having fleet angles. In a case where the guide pulleys 20 are supported directly by the first rotating shaft 16, the inner circumferential face 20d of each guide pulley 20 slides directly relative to the outer circumferential face of the first rotating shaft 16.

As illustrated in FIG. 8, the inner circumferential face 20d of each guide pulley 20 is formed so that the inner diameter d of the guide pulley 20 gradually increases from the position of a plane P, which is perpendicular to the center line CL and at which the inner diameter d is smallest, as the position is farther along the center line CL from the plane P. In other words, the inner circumferential face 20d may have a conical shape, an inverted cone shape, or a tapered shape. In addition, an inner circumferential face 20e opposite the inner circumferential face 20d with respect to the plane P is also processed to have a shape similar to the inner circumferential face 20d.

Note that each of the guide pulleys 20 according to the embodiment is formed such that the angle α between the inner circumferential face 20d or the inner circumferential face 20e and the center line CL is within a range from 3 to 7 degrees. In addition, one end face 20f of each guide pulley 20 is tilted at the angle α with respect to the plane P, and the angle between the end face 20f and the inner circumferential face 20d is 90°. Furthermore, the other end face 20g of the guide pulley 20 is parallel to the plane P (perpendicular to the center line CL). Each guide pulley 20 is therefore an annular member that is asymmetric with respect to the plane P.

As illustrated in FIGS. 5 and 6, the guide pulleys 20 according to the embodiment are arranged in such a manner that two guide pulleys 20 are arranged adjacent to each other on each side of the support 14 with the support 14 therebetween. In addition, as illustrated in FIG. 6, the end faces 20g of two guide pulleys 20 that are arranged adjacent to each other on the annular part 14a face each other. As a result, one (a guide pulley 20B) of two guide pulleys 20 that are adjacent to each other is in contact with and tilted with respect to the support 14, and the other (a guide pulley 20A) of the two guide pulleys 20 adjacent to each other is in contact with and tilted with respect to the base member 18. Thus, when a guide pulley 20 is tilted, the guide pulley 20 is accurately positioned in a first rotating shaft direction X.

As illustrated in FIG. 2, the guide pulleys 20 are each tilted with respect to the first rotating shaft 16 so that the wires 26 and 28 running over the guide pulleys 20 extend out toward the outer circumference of the jaw pulleys 24. As a result, even in a case where jaw pulleys 24 having a large diameter that are likely to increase the operation torque of the pair of grasping portions 12a and 12b are used, interference of wires at the guide pulleys 20 can be reduced.

Next, the base member 18 will be described. As illustrated in FIG. 2 and FIG. 5, the base member 18 according to the embodiment includes a pair of arms 18a and 18b that hold respective ends of the first rotating shaft 16, and third rotating shafts 36 that are held by the pair of arms 18a and 18b, respectively, and rotatably support four guide pulleys 34 located upstream of the guide pulleys 20. Note that a third rotating shaft 36 according to the embodiment is provided on each of the pair of arms 18a and 18b.

The arms 18a and 18b each have a base part 18c holding the third rotating shaft 36, and a distal end part 18d that holds the first rotating shaft 16 and that is thinner than the base part 18c. In other words, the distance between the distal end parts 18d is larger than the distance between the base parts 18c. Thus, as illustrated in FIG. 3, even when the wires 26 and 28 having the fleet angles are bent together with the support 14 around the first rotating shaft 16 (in the direction of an arrow F in FIG. 3), the wires 26 and 28 are less likely to interfere with the arms 18a and 18b.

Furthermore, the circumferential width W1 of the distal end part 18d of the arm 18a (the arm 18b) is smaller than the circumferential width W2 of the base part 18c thereof. Thus, a U-shaped recess is formed at the distal end part 18d at which interference with a wire needs to be addressed, and the circumferential width of the distal end part 18d is made larger than the circumferential width of the base part 18c, which minimizes deterioration of the stiffness of the arm.

In addition, as illustrated in FIG. 5, the jaw pulleys 24 according to the embodiment have an outer diameter G1, which is larger than the distance between the base parts 18c of the pair of arms 18a and 18b. Thus, in the forceps device 10 according to the embodiment, the jaw pulleys 24 having a large outer diameter relative to the inner diameter of the cylindrical base member 18 may be used.

### [Easiness of assembly of support]

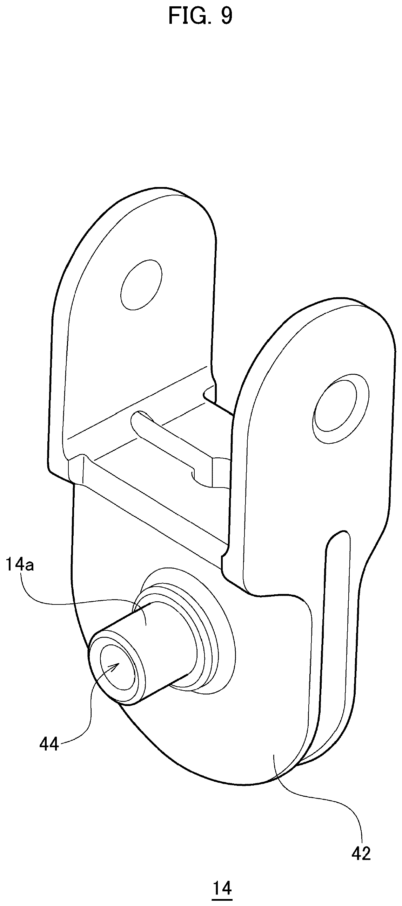

FIG. 9 is a perspective view of the support according to the embodiment. As illustrated in FIGS. 5 and 6, the forceps device 10 according to the embodiment includes the pair of grasping portions 12a and 12b, the support 14 that holds the pair of grasping portions 12a and 12b, the first rotating shaft 16 that turnably supports the support 14, the base member 18 that holds the first rotating shaft 16, and a plurality of guide pulleys 20 arranged coaxially with the first rotating shaft 16. As illustrated in FIG. 9, the support 14 has the annular part 14a, which is a cylindrical part through which the first rotating shaft 16 extends. The guide pulleys 20 are rotatably supported by the outer circumference of the annular part 14a. Note that each rotating shaft is not limited to a shaft that rotates by itself, but may be any shaft that is the center of rotation of a member supported thereby, and may be a shaft fixed to another member.

In the forceps device 10 having such a structure, the support 14 in a state in which a plurality of guide pulleys 20 are supported by the outer circumference of the annular part 14a is held by the base member 18 with the first rotating shaft 16 therebetween. This configuration facilitates improvement in the easiness of assembly as compared with a case where the support 14 is held directly by the base member 18.

In addition, the base member 18 of the embodiment has the pair of arms 18a and 18b facing each other. The first rotating shaft 16 is firmly fixed in such a manner that the axial ends thereof are press-fitted to the pair of arms 18a and 18b. As a result, because the distal ends of the pair of arms 18a and 18b, which can be free ends, are fixed by the rotating shaft, the stiffness of the whole base member 18 increases.

Each of the wires 26, 28, 30 and 32 transmits a driving force to the grasping portion 12a or the grasping portion 12b to move the grasping portion 12a or the grasping portion 12b. Specifically, the wire 26 and the wire 32 run over the jaw pulleys 24 for the grasping portion 12b, and the grasping portion 12b moves in an opening direction when the wire 26 is pulled and moves in a closing direction when the wire 32 is pulled. The wire 28 and the wire 30 run over the jaw pulleys 24 for the grasping portion 12a, and the grasping portion 12a moves in a closing direction when the wire 28 is pulled and moves in an opening direction when the wire 30 is pulled.

In addition, each of the four guide pulleys 20 has a corresponding one of the wires 26, 28, 30 and 32 placed thereover. Each of the wires 26, 28, 30 and 32 runs over the corresponding one of the guide pulleys 20 so that the normal force applied to the support 14 from the first rotating shaft 16 is reduced when the grasping portions 12a and 12b are moved. More specifically, as illustrated in FIG. 2, each of the wires 26, 28, 30 and 32 passes between the corresponding ones of the guide pulleys 20 and guide pulleys 34 and runs over the corresponding one of the guide pulleys 20 from a lower side thereof (a side opposite the grasping portion 12a or 12b with respect to the first rotating shaft 16) .

Thus, when tension is applied to at least any one of the wires 26, 28, 30 and 32 for moving the grasping portions 12a and 12b, the tension causes a force acting on the support 14 via the guide pulleys 20. In the case of the forceps device 10 according to the embodiment, the tension applied to the wires 26, 28, 30 and 32 causes a force acting on the support 14 in directions toward the grasping portions 12a and 12b.

The support 14 is formed integrally with a support pulley 42 over which the wires 38 and 40 for transmitting a driving force for rotating the support about the first rotating shaft 16 run. Thus, when one of the wires 38 and 40 is pulled to turn the support 14, the tension of the wire 38 or 40 running over the support pulley 42 presses the support 14 toward the first rotating shaft 16. A normal force received by the support 14 from the first rotating shaft 16 is thus generated, which contributes to an increase in frictional force.

In the forceps device 10 according to the embodiment, however, the tension of the wires 26, 28, 30 and 32 for moving the grasping portions 12a and 12b causes a force acting on the guide pulleys 20 in the upward direction in FIG. 2 as described above. The normal force caused by the wires 38 and 40 is reduced by the force acting on the guide pulleys 20, which reduces the frictional force when the support 14 turns about the first rotating shaft 16. Consequently, the movement of the grasping portions 12a and 12b becomes smoother, and the controllability of the forceps device 10 improves.

### [Method for producing forceps device]

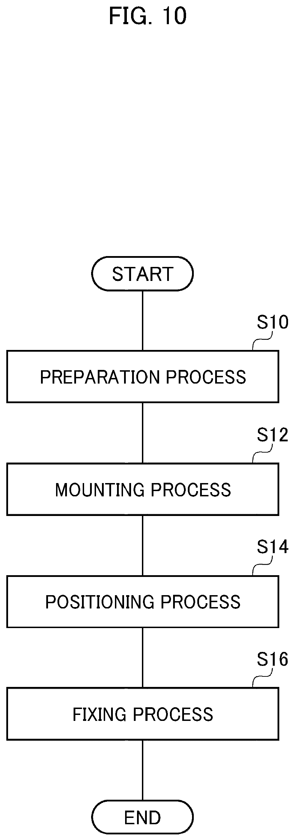

Next, a method for producing the forceps device 10 according to the embodiment will be explained. FIG. 10 is a flowchart explaining a method for manufacturing the forceps device according to the embodiment. In the method, the support 14 holding the grasping portions 12a and 12b is first prepared (S10). Subsequently, a plurality of guide pulleys 20 are mounted on the outer circumference of the annular part 14a through which a through-hole 44 (see FIG. 5) is formed (S12). The first rotating shaft 16 that turnably supports the support 14 is to be inserted in the through-hole 44. Subsequently, the support 14 is positioned so that holes 18e and 18f into which the first rotating shaft 16 is to be inserted and the through-hole 44 are linearly aligned with respect the base member 18 through which the holes 18e and 18f are formed (S14). The first rotating shaft 16 is then inserted into the hole 18e, and the respective ends of the first rotating shaft 16 are ultimately press-fitted into the holes 18e and 18f of the base member 18, so that the first rotating shaft 16 is firmly fixed to the base member 18 (S16). Note that a process of passing wires over the pulleys may be performed at a timing that does not interfere with the manufacture described above.

According to the manufacturing method, such a process as sandwiching a support by base member parts into which a base member is divided and then bonding the base member parts to each other need not be performed. In addition, such a process as coaxially arranging holes of a support, pulleys and a base member, and press-fitting a rotating shaft into the support while keeping the positions of the holes, which involves difficult adjustment, need not be performed.

### [Grasping portions]

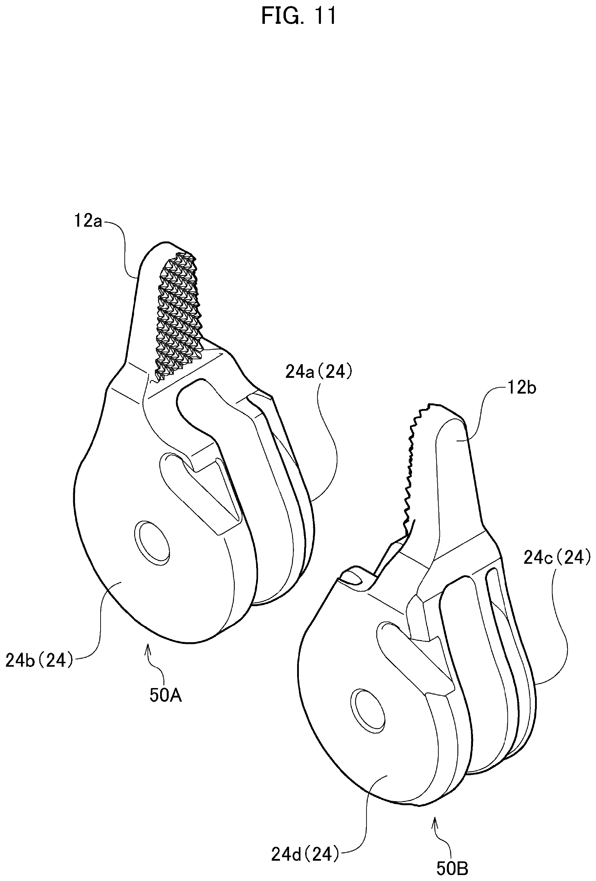

Next, jaw parts constituting the grasping portions according to the embodiment will be described in detail. FIG. 11 is a perspective view illustrating a state in which a pair of jaw parts facing each other are to be fitted according to the embodiment. Jaw parts 50A and 50B illustrated in FIG. 11 are parts having substantially the same shapes as each other. The jaw parts 50A and 50B have the grasping portion 12a and the grasping portion 12b, respectively, which move relative to each other to grasp an object. The grasping portion 12a is continuous with a jaw pulley 24a (first grasping portion pulley) and a jaw pulley 24b (second grasping portion pulley), which are formed in a bifurcated shape, and the grasping portion 12a and the jaw pulleys 24a and 24b integrally constitute the jaw part 50A. In addition, the grasping portion 12b is continuous with a jaw pulley 24c (third grasping portion pulley) and a jaw pulley 24d (fourth grasping portion pulley), which are formed in a bifurcated shape, and the grasping portion 12b and the jaw pulleys 24c and 24d integrally constitute the jaw part 50B.

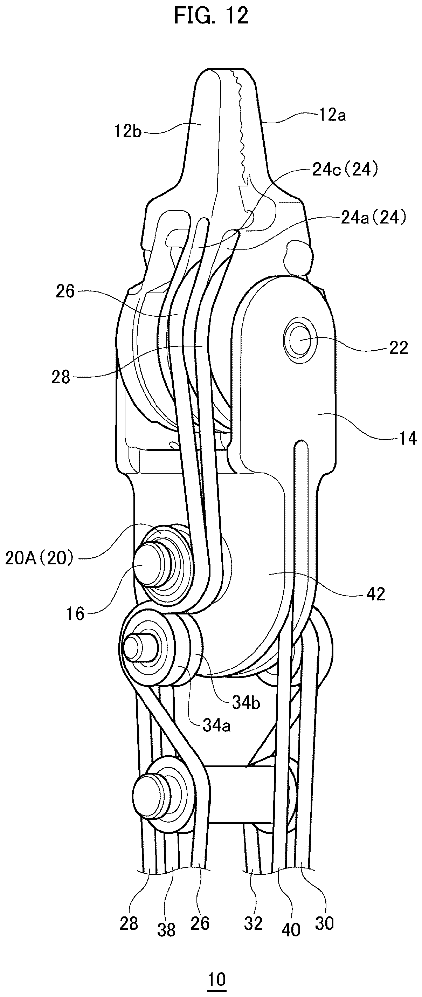

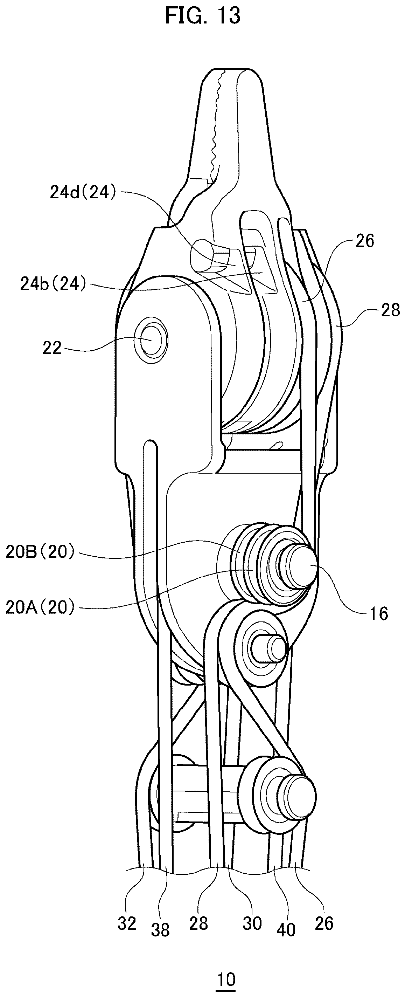

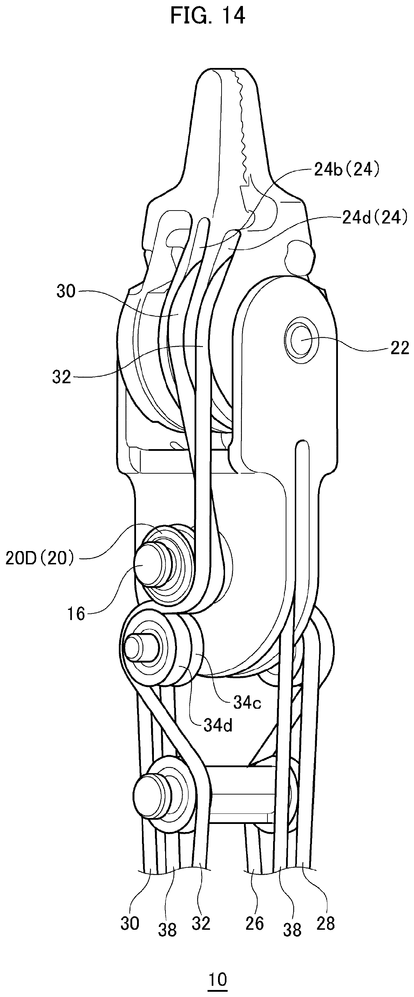

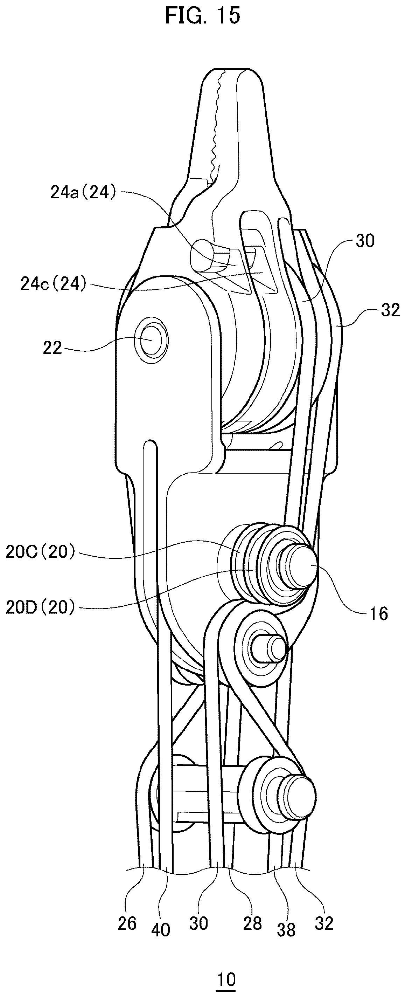

FIG. 12 is a perspective view of the forceps device 10 illustrated in FIG. 1 as viewed in a direction H1. FIG. 13 is a perspective view of the forceps device 10 illustrated in FIG. 1 as viewed in a direction H2. FIG. 14 is a perspective view of the forceps device 10 illustrated in FIG. 1 as viewed in a direction H3. FIG. 15 is a perspective view of the forceps device 10 illustrated in FIG. 1 as viewed in a direction opposite the direction H2. Note that the directions H1 to H3 are directions within a horizontal plane H including the first rotating shaft 16. In addition, the base member 18 is not illustrated in FIGS. 12 to 15.

As illustrated in FIGS. 12 to 15, the jaw pulley 24a, the jaw pulley 24c, the jaw pulley 24b, and the jaw pulley 24d are rotatably supported in this order by the second rotating shaft 22. In addition, the guide pulley 20A, the guide pulley 20B, the guide pulley 20C, and the guide pulley 20D are rotatably supported in this order by the first rotating shaft 16. The wires 26, 28, 30 and 32, which are grasping-portion wires for transmitting driving forces for causing the grasping portions 12a and 12b to perform opening and closing movements, run between the guide pulleys 20A to 20D and the grasping portions 12a and 12b without other pulleys. Note that the driving forces are input from an actuator unit outside of the forceps device 10. This configuration can shorten the distance between the first rotating shaft 16 and the second rotating shaft 22 as illustrated in FIG. 12, etc., and can therefore increase the torque (operation force) of the grasping portions 12a and 12b. Consequently, the forceps device 10 with high controllability of the grasping portions 12a and 12b can be provided.

### [How wires run over pulleys]

Next, the manner in which the wires 26, 28, 30 and 32 according to the embodiment run over the pulleys will be described in detail. The wire 26 runs between the guide pulley 20A and the jaw pulley 24c. The wire 28 runs between the guide pulley 20B and the jaw pulley 24a. The wire 30 runs between the guide pulley 20C and the jaw pulley 24b. The wire 32 runs between the guide pulley 20D and the jaw pulley 24d.

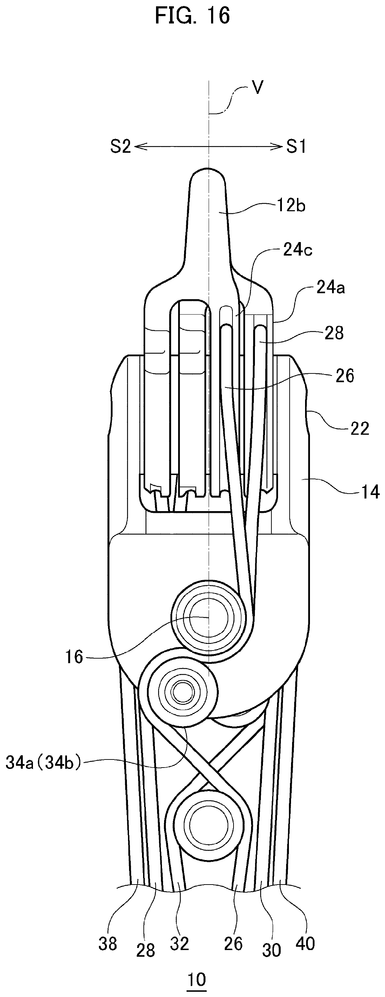

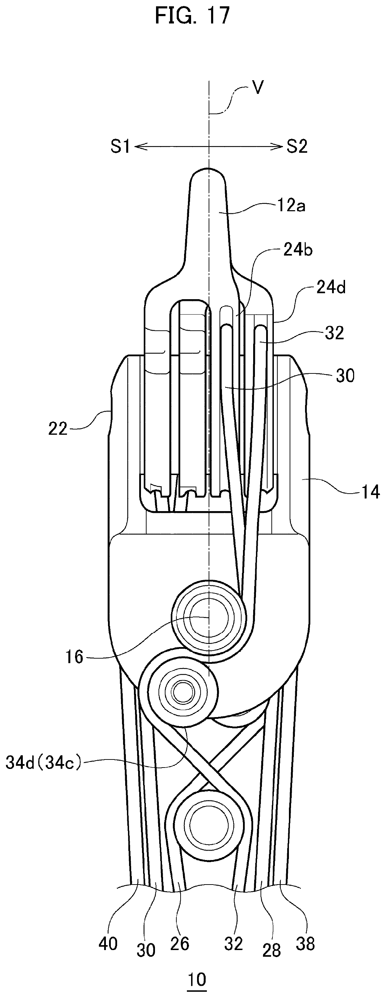

FIG. 16 is a view of the forceps device 10 illustrated in FIG. 3 without illustration of the base member 18. FIG. 17 is a view of the forceps device 10 illustrated in FIG. 4 without illustration of the base member.

As illustrated in FIGS. 12, 13, and 16, the wire 26 is passed over a first side S1 of the guide pulley 20A with respect to a vertical cross section V including the first rotating shaft 16 and being perpendicular to the second rotating shaft 22, and then fixed to the jaw pulley 24c located on the first side S1 with respect to the vertical cross section V. The wire 28 is passed over the first side S1 of the guide pulley 20B with respect to the vertical cross section V, and then fixed to the jaw pulley 24a located on the first side S1 with respect to the vertical cross section V.

In addition, as illustrated in FIGS. 14 to 16, the wire 30 is passed over the second side S2 of the guide pulley 20C with respect to the vertical cross section V, and then fixed to the jaw pulley 24b located on the second side with respect to the vertical cross section V. The wire 32 is passed over the second side S2 of the guide pulley 20D with respect to the vertical cross section V, and then fixed to the jaw pulley 24d located on the second side S2 with respect to the vertical cross section V. As a result, the four grasping-portion wires are fixed to the four grasping portion pulleys, respectively, without intersecting with each other. In addition, the four grasping-portion wires running over the guide pulleys 20A to 20D are fixed to the four grasping portion pulleys, respectively, with small fleet angles.

Furthermore, the forceps device 10 includes the guide pulley 34a on the upstream side of the guide pulley 20A (on the side opposite the grasping portions) and on the second side S2 with respect to the vertical cross section V, the guide pulley 34b on the upstream side of the guide pulley 20B and on the second side S2 with respect to the vertical cross section V, the guide pulley 34c on the upstream side of the guide pulley 20C and on the first side S1 with respect to the vertical cross section V, and the guide pulley 34d on the upstream side of the guide pulley 20D and on the first side S1 with respect to the vertical cross section V.

As a result, when the support 14 is bent in either direction about the first rotating shaft 16, one or more of the wires 26, 28, 30 and 32 come in contact with the associated one or more of the guide pulleys 20A to 20D, which stabilizes the controllability when the support 14 is turned. More specifically, when the support 14 is bent from the state illustrated in FIG. 16 toward the second side S2, the wires 26 and 28 come in contact with the guide pulleys 20A and 20B, respectively. In addition, when the support 14 is bent from the state illustrated in FIG. 17 toward the first side S1, the wires 30 and 32 come in contact with the guide pulleys 20C and 20D, respectively. Thus, in either case, such a state in which none of the wires are in contact with the guide pulleys 20A to 20D (run over the guide pulleys 20A to 20D) is avoided. As a result, the controllability when the support 14 is bent about the first rotating shaft 16 is stabilized.

### [Sealing of base member]

Because the grasping portions 12a and 12b of the forceps device 10 according to the embodiment are operated inside the abdominal cavity of a patient, it is necessary to devise a way to prevent gas around the grasping portions 12a and 12b from leaking out through the forceps device 10 so that the air pressure in the abdominal cavity, which is increased to be higher than the atmospheric pressure, does not lower. In the present embodiment, sealing is therefore provided in a region including the base member 18.

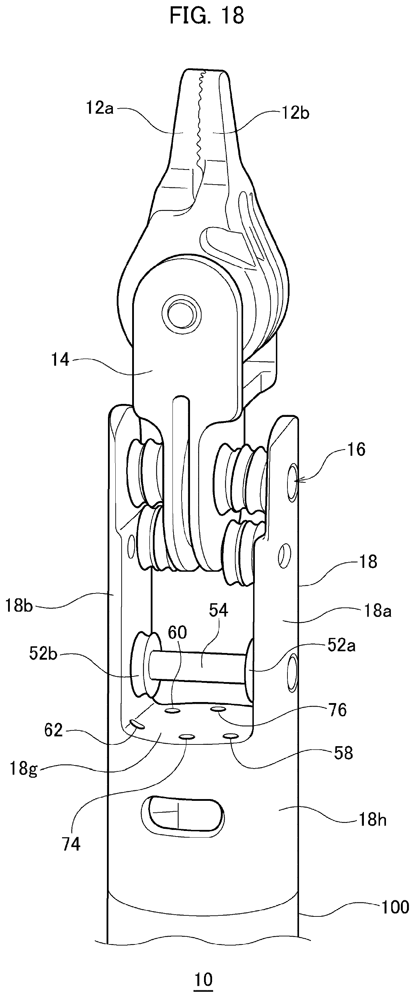

FIG. 18 is a view of the forceps device illustrated in FIG. 1 without illustration of wires. As illustrated in FIG. 18, the forceps device 10 includes the pair of grasping portions 12a and 12b, the support 14 that holds the pair of grasping portions 12a and 12b, the first rotating shaft 16 that turnably supports the support 14, the base member 18 that holds the first rotating shaft 16, a plurality of wires 26, 28, 30 and 32 (not illustrated in FIG. 18; see FIG. 2) for transmitting driving forces to the pair of grasping portions 12a and 12b to move the grasping portions 12a and 12b, a guide pulley 52a for guiding the wire 26, a guide pulley 52b for guiding the wire 32, and a support shaft 54 that rotatably supports the guide pulleys 52a and 52b. The base member 18 is located between the grasping portions 12a and 12b and a shaft 100 of the forceps device 10.

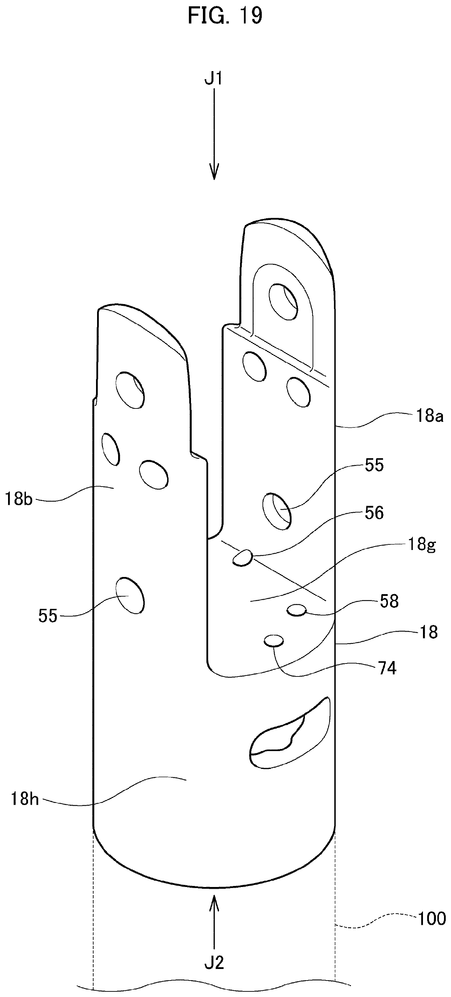

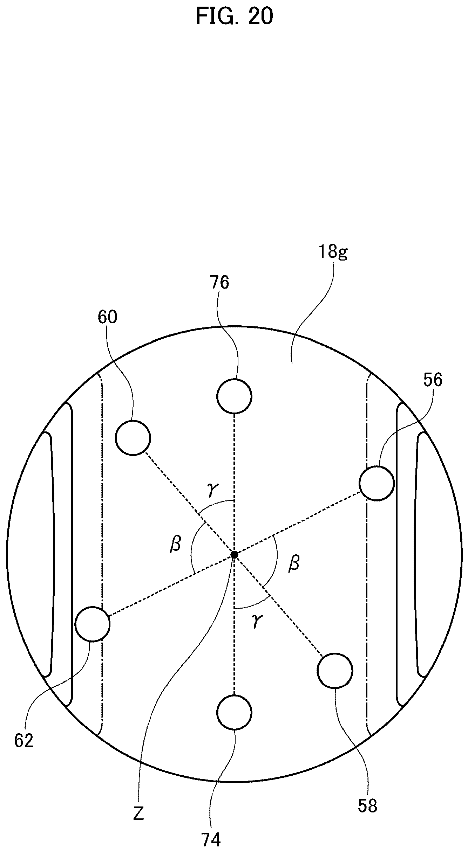

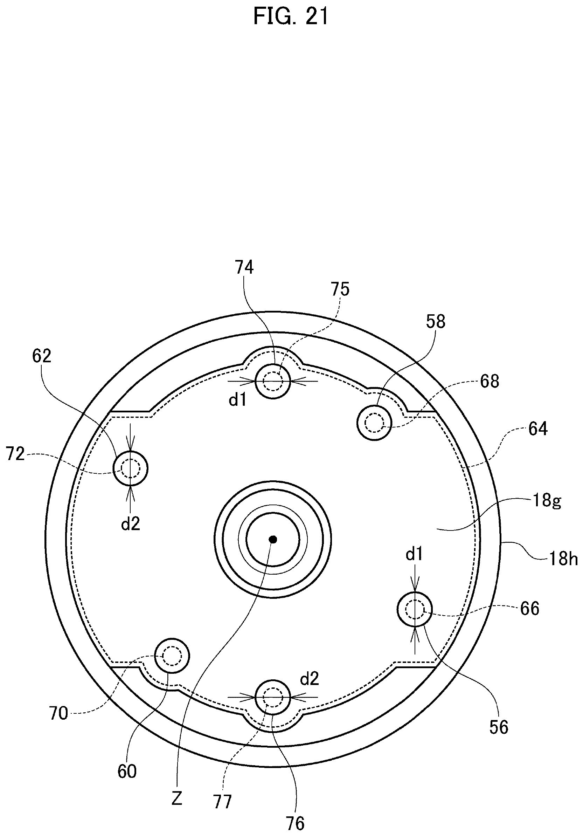

FIG. 19 is a perspective view of the base member according to the embodiment. FIG. 20 is a top view of the base member illustrated in FIG. 19 as viewed in a direction J1. FIG. 21 is a bottom view of the base member illustrated in FIG. 19 as viewed in a direction J2.

The base member 18 includes a partition part 18g having a plurality of grasping-portion-wire holes 56, 58, 60 and 62 through which the wires 26, 28, 30 and 32, respectively, pass, and the pair of arms 18a and 18b which extend from an outer edge of the partition part 18g toward the grasping part (upward in FIG. 19) and to which respective ends of the support shaft 54 are fixed. The arms 18a and 18b each have a fitting hole 55 into which an end of the support shaft 54 that rotatably supports the pair of guide pulleys 52a and 52b is fitted. In this manner, the pair of arms 18a and 18b are fixed via the support shaft 54, which increases the stiffness of the base member 18.

The partition part 18g is a partition formed between a cylindrical part 18h and the pair of arms 18a and 18b. The grasping-portion-wire holes 56 and 58 are a pair of grasping-portion-wire holes that are adjacent to each other at the closet distance, and the grasping-portion-wire holes 60 and 62 are a pair of grasping-portion-wire holes that are adjacent to each other at the closest distance.

As illustrated in FIG. 12, the wires 26 and 28 in a state being guided by the guide pulleys 34a and 34b are arranged together. Thus, for passing the wires 26 and 28 in this state through the partition part 18g, a large hole through which two wires can pass needs to be formed through the partition part 18g or two small holes through each of which one wire can pass need to be formed through the partition part 18g. In the case of a large hole, the airtightness of sealing with sealing resin, which will be described later, may be lowered. In contrast, in the case of two adjacent small holes, the wall between the holes becomes thin, and the strength of an area of the base member 18 where the holes are formed may therefore be lowered.

Hence, in the forceps device 10 according to the embodiment, the wire 26 is guided by the guide pulley 52a, so that the wire 26 is separated from the wire 28. Similarly, the wire 32 is guided by the guide pulley 52b, so that the wire 32 is separated from the wire 30. In this manner, the grasping-portion-wire hole 56 through which the wire 26 passes and the grasping-portion-wire hole 58 through which the wire 28 passes can be separated from each other. Similarly, the grasping-portion-wire hole 60 through which the wire 30 passes and the grasping-portion-wire hole 62 through which the wire 32 passes can be separated from each other.

In addition, as illustrated in FIG. 21, the base member 18 includes an elastic sealing member 64 (having a type A durometer hardness of 30 to 70) arranged on one side (on one side opposite the side in which the grasping portions 12a and 12b are located) with respect to the partition part 18g. The sealing member 64 is a silicone resin film having a thickness of about 0.5 to 2 mm, for example.

The sealing member 64 illustrated in FIG. 21 has a plurality of sealing holes 66, 68, 70, and 72 at positions corresponding to those of the grasping-portion-wire holes 56, 58, 60 and 62. This enables sealing with the sealing member 64, with the wires 26, 28, 30 and 32 passing through the partition part 18g of the base member 18. The wires have a diameter φ of 0.4 to 0.5 mm, and the diameter d1 of the grasping-portion-wire holes is preferably slightly larger than the wire diameter.

The sealing holes have a diameter d2 that is smaller than the diameter d1 of the grasping-portion-wire holes and smaller than the diameter (wire diameter φ) of the grasping-portion wires. As a result, even when a gap is present between a grasping-portion-wire hole and a grasping-portion wire, the sealing hole and the grasping-portion wire are in close contact with each other, which reduces leakage of gas from the grasping part side to the outside of the forceps device 10 via the base member 18.

As illustrated in the drawings, the four grasping-portion-wire holes according to the embodiment are formed through the partition part 18g at certain distances from each other. Thus, the sealing holes formed at positions corresponding to those of the grasping-portion-wire holes are also separated from the adjacent sealing holes.

In addition, the forceps device 10 according to the embodiment includes the pair of wires 38 and 40 for rotating the support 14 about the first rotating shaft 16 in addition to the wires 26, 28, 30 and 32, which are grasping-portion wires. The partition part 18g of the base member 18 therefore has support-wire holes 74 and 76 through which the wires 38 and 40, respectively, pass. The support-wire holes 74 and 76 are formed at point-symmetric positions with respect to the center Z of the partition part 18g (the central axis of the cylindrical part 18h).

Furthermore, the sealing member 64 has a plurality of sealing holes 75 and 77 formed at positions corresponding to those of the support-wire holes 74 and 76. This enables sealing with the sealing member 64, with the wires 38 and 40 passing through the partition part 18g of the base member 18. The diameter d1 of the support-wire holes is preferably slightly larger than the wire diameter.

### [Method for producing sealing member]

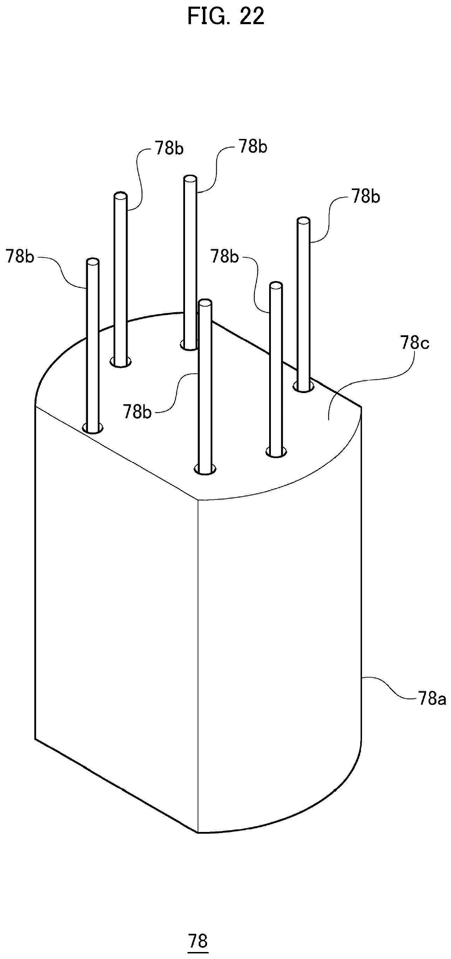

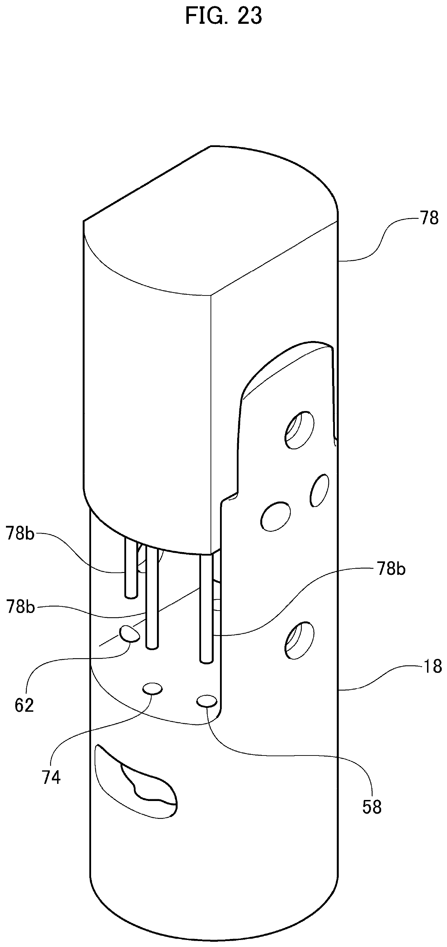

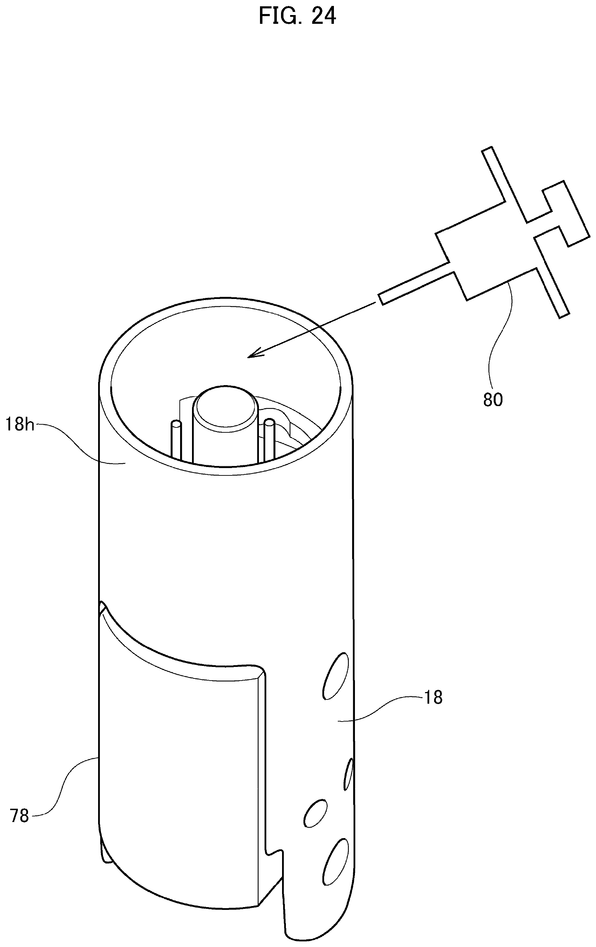

Next, a method for producing the sealing member 64 will be explained. FIG. 22 is a perspective view of a jig to be used for producing a sealing member. FIG. 23 is a perspective view illustrating a state in which the jig illustrated in FIG. 22 is attached to the base member. FIG. 24 is a schematic diagram illustrating injection of sealing resin in a state in which the jig is attached to the base member.

The jig 78 illustrated in FIG. 22 includes a columnar body 78a inserted between the pair of arms 18a and 18b of the base member 18, and six shafts 78b extending from one end face 78c of the body 78a. The six shafts 78b are arranged at predetermined positions on the one end face 78c of the body 78a so that the shafts 78b are inserted into the four grasping-portion-wire holes 56, 58, 60 and 62 and the two support-wire holes 74 and 76 of the partition part 18g.

As illustrated in FIG. 23, the jig 78 is attached between the arms 18a and 18b of the base member 18, and an injection device 80 such as an injector is used to inject sealing resin through an opening of the cylindrical part 18h in a state in which the shafts 78b stick out from a rear face of the partition part 18g. After the sealing resin has hardened and the sealing member 64 is thus molded, the jig 78 is removed, and the sealing holes 66, 68, 70, 72, 75 and 77 are thus formed.

Because the partition part 18g according to the embodiment has six holes formed at dispersed positions, the six shafts 78b of the jig 78 are also arranged at dispersed positions. Thus, the spaces between adjacent shafts 78b are large, and sealing resin injected by the injection device 80 smoothly enters regions between the adjacent shafts 78b. In contrast, if the spaces between adjacent shafts 78b are narrow, there may be cases where viscous sealing resin that has been injected does not smoothly enter regions between adjacent shafts 78b. In such a case, adjacent sealing holes may be integrated, or a sealing hole may not have a predetermined shape.

In the base member 18 according to the embodiment, the holes through the partition part 18g are therefore formed at dispersed positions away from each other so that the sealing holes in the molded sealing member 64 are not close to each other. More specifically, as illustrated in FIG. 20, when the partition part 18g is viewed in the vertical direction, an angle β at the center Z of the partition part 18g between the grasping-portion-wire hole 56 and the grasping-portion-wire hole 58 is equal to or larger than 30° or, more preferably, equal to or larger than 45°, for example.

As a result, the distance between the grasping-portion-wire hole 56 and the grasping-portion-wire hole 58 becomes large, which reduces such defects as integration of holes when the sealing member 64 with a plurality of sealing holes is molded. In other words, the sealing member 64 having the sealing holes is molded with high accuracy on the rear face side of the partition part 18g having the grasping-portion-wire holes and the support-wire holes, and the airtightness on the grasping part side is increased.

Furthermore, when the partition part 18g is viewed in the vertical direction, an angle γ at the center Z of the partition part 18g between the support-wire hole 74 and the grasping-portion-wire hole 58 that is closest to the support-wire hole 74 is equal to or larger than 30° or, more preferably, equal to or larger than 35°, for example. As a result, the support-wire hole 74 and the grasping-portion-wire hole 58 can be separated from each other. Thus, the sealing member 64 having the sealing holes is molded with high accuracy on the rear face side of the partition part 18g having the grasping-portion-wire holes and the support-wire holes, and the airtightness on the grasping part side is increased.

### Industrial Applicability

The present invention can be used for a manipulator of a surgical robot.

### Reference Signs List

10 forceps device
12a, 12b grasping portion
14 support
16 first rotating shaft
18 base member
18a, 18b arm
18g partition part
26, 28, 30, 32 wire
38, 40 wire
52a, 52b guide pulley
54 support shaft
56, 58, 60, 62 grasping-portion-wire hole
64 sealing member
66 sealing hole
74, 76 support-wire hole

## Claims

1. A forceps device (10) comprising:
a pair of grasping portions (12a, 12b);
a support (14) that holds the pair of grasping portions (12a, 12b);
a rotating shaft (16) that turnably supports the support (14);
a base member (18) that holds the rotating shaft (16);
a plurality of grasping-portion wires (26, 28, 30, 32) for transmitting driving forces to cause the pair of grasping portions (12a, 12b) to perform opening and closing movements;
guide pulleys (34a, 34b, 34c, 34d, 52a, 52b) for guiding one or some of the plurality of grasping-portion wires (26, 28, 30, 32); and
a support shaft (54) for rotatably supporting the guide pulley (52a, 52b), wherein
the base member (18) includes:
a partition part (18g) having a plurality of grasping-portion-wire holes (56, 58, 60, 62) through which the respective grasping-portion wires (26, 28, 30, 32) pass; and
a pair of arms (18a, 18b) extending from the partition part (18g) toward the pair of grasping portions (12a, 12b), respective ends of the support shaft (54) being fixed to the respective arms (18a, 18b),
the plurality of grasping-portion wires (26, 28, 30, 32) includes a first grasping-portion wire and a second grasping-portion wire, wherein
the plurality of grasping-portion-wire holes includes a first grasping-portion-wire hole through which the first grasping-portion wire passes, and a second grasping-portion-wire hole which is closest to the first grasping-portion-wire hole and through which the second grasping-portion wire passes, and
the guide pulleys (34a, 34b, 34c, 34d, 52a, 52b) include
a first guide pulley (34a, 34b, 34c, 34d) that guides the first grasping-portion wire (26, 32) and the second grasping-portion wire (28, 30) together; and
a second guide pulley (52a, 52b) that is located upstream of the first guide pulley (34a, 34b, 34c, 34d) and guides one or both of the first grasping-portion wire (26, 32) and the second grasping-portion wire (28, 30) so that the first grasping-portion wire (26, 32) is moved away from the second grasping-portion wire (28, 30).

2. The forceps device according to claim 1, wherein
when the partition part (18g) is viewed in a vertical direction, an angle at a center of the partition part (18g) between the first grasping-portion-wire hole and the second grasping-portion-wire hole is equal to or larger than 30°.

3. The forceps device according to claim 1 or 2, wherein
the base member (18) includes a sealing member (64) on one side with respect to the partition part (18g), and
the sealing member (64) has a plurality of sealing holes (66, 68, 70, 72) at positions corresponding to the plurality of grasping-portion-wire holes (56, 58, 60, 62).

4. The forceps device according to claim 3, wherein
the sealing holes (66, 68, 70, 72) each have a diameter smaller than a diameter of a corresponding one of the grasping-portion-wire holes (56, 58, 60, 62) and smaller than a diameter of a corresponding one of the grasping-portion wires (26, 28, 30, 32).

5. The forceps device according to claim 3 or 4, wherein the sealing member (64) is a silicone resin film.

6. The forceps device according to any one of claims 1 to 5, further comprising:
a pair of support wires (38, 40) for transmitting driving forces to turn the support, wherein
the partition part (18g) has a pair of support-wire holes (74, 76) through which the pair of support wires (38, 40), respectively, pass, and
when the partition part (18g) is viewed in a vertical direction, an angle at a center of the partition part (18g) between each of the support-wire holes (74, 76) and the grasping-portion-wire hole that is closest to the support-wire hole is equal to or larger than 30°.

## Patentansprüche

1. Zangenvorrichtung (10), enthaltend:
ein Paar Greifabschnitte (12a, 12b);
eine Halterung (14), die das Paar Greifabschnitte (12a, 12b) hält;
eine Rotationswelle (16), die die Halterung drehbar hält (14);
ein Basiselement (18), das die Rotationswelle hält (16);
eine Vielzahl an Greifabschnittsdrähten (26, 28, 30, 32) zur Übertragung von Treibkräften, um das Paar Greifabschnitte (12a, 12b) zu veranlassen, Öffnungs- und Schließbewegungen auszuführen;
Führungsrollen (34a, 34b, 34c, 34d, 52a, 52b) zur Führung eines oder mehrerer der Vielzahl an Greifabschnittsdrähten (26, 28, 30, 32); und
eine Halterungswelle (54) zum drehbaren Halten der Führungsrolle (52a, 52b), wobei
das Basiselement (18) einschließt:
ein Trennungsteil (18g) mit einer Vielzahl an Greifabschnittsdrahtlöchern (56, 58, 60, 62), durch die die jeweiligen Greifabschnittsdrähte (26, 28, 30, 32) laufen; und
ein Paar Arme (18a, 18b), die sich von dem Trennungsteil (18g) in Richtung zu dem Paar Greifabschnitte (12a, 12b) erstrecken, wobei die jeweiligen Enden der Halterungswelle (54) an den jeweiligen Armen befestigt sind (18a, 18b),
die Vielzahl an Greifabschnittsdrähten (26, 28, 30, 32) einen ersten Greifabschnittsdraht und einen zweiten Greifabschnittsdraht einschließen, wobei
die Vielzahl an Greifabschnittsdrahtlöchern ein erstes Greifabschnittsdrahtloch, durch das der erste Greifabschnittsdraht läuft, und ein zweites Greifabschnittsdrahtloch einschließt, das dem ersten Greifabschnittsdrahtloch am nächsten ist und durch das der zweite Greifabschnittsdraht läuft, und
die Führungsrollen (34a, 34b, 34c, 34d, 52a, 52b)
eine erste Führungsrolle (34A, 34B, 34C, 34D), die den ersten Greifabschnittsdraht (26, 32) und den zweiten Greifabschnittsdraht (28, 30) zusammen führt; und
eine zweite Führungsrolle (52A, 52B) einschließen, die stromaufwärts der ersten Führungsrolle (34A, 34B, 34C, 34D) positioniert ist und einen oder beide des ersten Greifabschnittdrahts (26, 32) und des zweiten Greifabschnittsdrahts (28, 30) führt, sodass der erste Greifabschnittsdraht (26, 32) von dem zweiten Greifabschnittsdraht (28, 30) wegbewegt wird.

2. Zangenvorrichtung gemäß Anspruch 1, wobei
wenn das Trennungsteil (18g) in eine senkrechte Richtung betrachtet wird, ein Winkel an einem Zentrum des Trennungsteils (18g) zwischen dem ersten Greifabschnittsdrahtloch und dem zweiten Greifabschnittsdrahtloch gleich oder größer als 30° ist.

3. Zangenvorrichtung gemäß Anspruch 1 oder 2, wobei
das Basiselement (18) ein Dichtungselement (64) auf einer Seite gegenüber dem Trennungsteil (18g) enthält, und
das Dichtungselement (64) eine Vielzahl von Dichtungslöchern (66, 68, 70, 72) an Positionen aufweist, die der Vielzahl an Greifabschnittdrahtlöchern entsprechen (56, 58, 60, 62).

4. Zangenvorrichtung gemäß Anspruch 3, wobei
die Dichtungslöcher (66, 68, 70, 72) jeweils einen Durchmesser aufweisen, der kleiner ist als ein Durchmesser eines entsprechenden der Greifabschnittdrahtlöcher (56, 58, 60, 62) und kleiner ist als ein Durchmesser eines entsprechenden der Greifabschnittdrähte (26, 28, 30, 32).

5. Zangenvorrichtung gemäß Anspruch 3 oder 4, wobei
das Dichtungselement (64) eine Silikonharzschicht ist.

6. Zangenvorrichtung gemäß einer der Ansprüche 1 bis 5, weiterhin enthaltend:
ein Paar Halterungsdrähte (38, 40) zur Übertragung von Treibkräften zur Drehung der Halterung, wobei
das Trennungsteil (18G) ein Paar Halterungsdrahtlöcher (74, 76) aufweist, durch die das Paar Halterungsdrähte (38, 40) jeweils läuft, und
wenn das Trennungsteil (18g) in eine senkrechte Richtung betrachtet wird, ein Winkel an einem Zentrum des Trennungsteils (18g) zwischen jedem der Halterungsdrahtlöcher (74, 76) und dem Greifabschnittsdrahtloch, das dem Halterungsdrahtloch am nächsten ist, gleich oder größer 30° ist.

## Revendications

1. Un dispositif de forceps (10) comprenant :
une paire de portions de préhension (12a, 12b) ;
un support (14) qui supporte la paire de portions de préhension (12a, 12b) ;
un arbre rotatif (16) qui soutient tournablement le support (14) ;
un élément de base (18) qui supporte l'arbre rotatif (16) ;
une pluralité de fils de portion de préhension (26, 28, 30, 32) pour transmettre les forces motrices afin de faire la paire de portions de préhension (12A, 12B) exécuter les mouvements d'ouverture et de fermeture;
des poulies guides (34a, 34b, 34c, 34d, 52a, 52b) pour guider un ou plusieurs de la pluralité des fils de portion de préhension (26, 28, 30, 32) ; et
un arbre de support (54) pour supporter de façon rotative la poulie guide (52A, 52B), dans lequel
l'élément de base (18) inclut :
une partie de séparation (18g) comportant une pluralité de trous pour les fils de portion de préhension (56, 58, 60, 62) par lesquels passent les fils de portion de préhension respectifs (26, 28, 30, 32) ; et
une paire de bras (18a, 18b) s'étendant de la partie de séparation (18g) vers la paire de portions de préhension (12a, 12b), les extrémités respectives de l'arbre de support (54) étant fixées aux bras respectifs (18a, 18b),
la pluralité des fils de portion de préhension (26, 28, 30, 32) inclut un premier fil de portion de préhension et un second fil de portion de préhension, dans lequel
la pluralité des trous de fil de portion de préhension comprend un premier trou de fil de portion de préhension par lequel passe le premier fil de portion de préhension, et un second trou de fil de portion de préhension qui est le plus proche du premier trou de fil de portion de préhension et par lequel passe le second fil de portion de préhension, et
les poulies guides (34A, 34B, 34C, 34D, 52a, 52b) incluent
une première poulie guide (34A, 34B, 34C, 34D) qui guide ensemble le premier fil de portion de préhension (26, 32) et le second fil de portion de préhension (28, 30) ; et
une seconde poulie guide (52A, 52B) située en amont de la première poulie guide (34A, 34B, 34C, 34D) et guide un ou les deux fils du premier fil de portion de préhension (26, 32) ainsi que du second fil de portion de préhension (28, 30) de sorte que le premier fil de portion de préhension (26, 32) soit déplacé loin du second fil de portion de préhension (28, 30).

2. Le dispositif de forceps selon la revendication 1, dans lequel
lorsque la partie de séparation (18g) est vue dans un sens vertical, un angle à un centre de la partie de séparation (18g) entre le premier trou de fil de portion de préhension et le second trou de fil de portion de préhension est égal ou supérieur à 30°.

3. Le dispositif de forceps selon la revendication 1 ou 2, dans lequel
l'élément de base (18) inclut un élément d'étanchéité (64) d'un côté par rapport à la partie de séparation (18g), et
l'élément d'étanchéité (64) possède une pluralité de trous d'étanchéité (66, 68, 70, 72) à des positions correspondant à la pluralité de trous de fil de portion de préhension (56, 58, 60, 62) .

4. Le dispositif de forceps selon la revendication 3, dans lequel
les trous d'étanchéité (66, 68, 70, 72) ont chacun un diamètre inférieur à un diamètre d'un correspondant des trous de fil de portion de préhension (56, 58, 60, 62) et inférieur un diamètre d'un correspondant des fils de portion de préhension (26, 28, 30, 32).

5. Le dispositif de forceps selon la revendication 3 ou 4, dans lequel
l'élément d'étanchéité (64) est un film en résine silicone.

6. Le dispositif de forceps selon l'une des revendications 1 à 5, comprenant en outre :
une paire de fils de support (38, 40) pour transmettre des forces motrices afin de faire tourner le support, dans lequel
la partie de séparation (18G) possède une paire de trous de fil de support (74, 76) par lesquels la paire de fils de support (38, 40) passent, respectivement, et
lorsque la partie de séparation (18G) est vue dans un sens vertical, un angle au centre de la partie de séparation (18G) entre chacun des trous de fil de support (74, 76) et le trou de fil de portion de préhension le plus proche du trou de fil de support est égal ou supérieur à 30°.
